# EUROPEAN PATENT APPLICATION

(11) **EP 0 884 382 A2**
(43) Date of publication of application: **16.12.1998**
(21) Application number: 98304379.5
(22) Date of filing: 03.06.1998
(51) Int. Cl.: C12N 7/00, C07K 14/055, C12N 5/06, C12N 5/10, A61K 39/255, C12N 15/85, C12N 15/38

(54) **Processes for preparation of marek's disease virus using continuous mammalian cell lines**

(30) Priority: 10.06.1997 US 49055 P
(71) Applicant: Pfizer Products Inc., Groton, CT 06340-5146 (US)
(72) Inventor: Brown, Tracy Michelle, Ashaway, Rhode Island 02804 (US); Calvert, Jay Gregory, Gales Ferry, Connecticut 06335 (US); Deichert, Michael Clayton, Lincoln, Nebraska 68504 (US); May, Stephen Wayne, Lincoln, Nebraska 68516 (US); Mulkey, Kent Michael, Salem, Connecticut 06420 (US); O'Hara, Michael Kenneth, East Lyme, Connecticut 06333 (US); Rong, Sing, Old Lyme, Connecticut 06371 (US); Sheppard, Michael George, North Stonington, Connecticut 06359 (US)
(74) Representative: Hayles, James Richard

(57) **Abstract**

The present invention relates to the use of continuous mammalian cell lines for the production of non-recombinant and recombinant Marek's Disease Virus, the production of genetically engineered Marek's Disease Virus and Marek's Disease Virus vectors, continuous mammalian cell lines infected with Marek's Disease Virus, Marek's Disease Virus vaccines produced using Marek's Disease Virus obtained using such processes and methods for protecting animals, such as avians, against disease by Marek's Disease Virus and/or other pathogens. Particularly, the mammalian cell lines capable of infection or transfection with MDV are feline kidney cell lines.

### Annex to the description

SEQUENCE LISTING

(1) GENERAL INFORMATION:

(i) APPLICANT: Pfizer Inc

(ii) TITLE OF INVENTION: PROCESS FOR PREPARATION OF MAREK'S
DISEASE VIRUS USING CONTINUOUS MAMMALIAN CELL LINES

(iii) NUMBER OF SEQUENCES: 2

(iv) CORRESPONDENCE ADDRESS:
(A) ADDRESSEE: Pfizer Inc
(B) STREET: 235 East 42nd Street
(C) CITY: New York
(D) STATE: New York
(E) COUNTRY: United States of America
(F) ZIP: 10017

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vi) CURRENT APPLICATION DATA:
(A) APPLICATION NUMBER: US 60/049,055
(B) FILING DATE: 10-JUN-1997
(C) CLASSIFICATION:

(viii) ATTORNEY/AGENT INFORMATION:
(A) NAME: Ling, Lorraine B.
(B) REGISTRATION NUMBER: 35,251
(C) REFERENCE/DOCKET NUMBER: PC9888

(ix) TELECOMMUNICATION INFORMATION:
(A) TELEPHONE: 212-573-2030
(B) TELEFAX: 212-309-4801

(2) INFORMATION FOR SEQ ID NO:1:

(i) SEQUENCE CHARACTERISTICS:
(A) LENGTH: 2023 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:1:
ACGGTCTCTG AACAAGACGG GCGATAATAT TAGCCATGTT TCGCATAGCC GTACCTCCCG 60
TTCTCTCCTG ATTATTTGAA AATGATAAAG TAGCCGTTTT ATTACAAGCT ATATGATTCC 120
TCAAATCCGT TACGTTAGCA GACGCCTTTC CACTGCGTCG TTGTATATGT ATCGTGTTTG 180
TATTATGACG TTTTAAAATT TTATGAGTGT CAGTTATCCG TGCTTTATAG TCAGACGCGG 240
TCGCCAATAT AGAGCATAGT CTATGAAAAT CAGTCACTAT GTGCCTTTTC TTTAGGCACA 300
TCACATGTAG AACAGACAGT TTTCGTCTTG CTACAAATAC TAACATTGGA CAAATAACGA 360
TACAATCTGA TCCTTGAGGC GCAATTTGCC CAATCAGAGA TTTGGAATCC AATAACTGCT 420
TTATGCCGGT GAGTCTTTGT TCATGTTTAC TGCGTGTCTT CAGGTTACGA GAAAATTTGC 480
AAGTTTTTAG TTCTAGAATG ACGCATACTC CATCACAGCC TACTTCCCAC AAATCACGAG 540
GCAACTTAAA CATGCAAATA CAATCCGGTC TACGTCGTTC TAGGTTTACT TCGAAGACCA 600
ATCGAAAATC CGTCAACTGT TTAAATACAT CTAATACCAT GACCTTCCCA AAAATTTTGG 660
CAAAGCTTCT CCCCGGCCAA TCATACACCT GAGATCCTAG ACACATCGCT TCTGCATAAA 720
GCCGTTTGTA AAAGCGATCG TGACATCGAA CACCAGCCGC TAAACGTCGC TTTCTAAGGA 780
CATTCGTATT TACATGCCGT TTGAAATTTC GAGTGCTACT AACCTGTCTG CGATATCTTT 840
TGAGTACGTT CTTCTCTCCC ATTGAACATG TCGGAGCCAC AATCGTGGTC GGTAATGGCA 900
TCTCAGATGA CATCTGCACA GCTCATACGT GTATACCTCG ATGGATCAAT GGGTATAGGT 960
AAAACGTCAA TGTTGAATGA GATACCGACG CACTCTTTAA TGGGAGTACC CGTACTAAAG 1020
GTTTTCGAAC CTATGAAATA CTGGCGGTAT TATTTTACTG ATTTGGTCAC GACCGTAAAT 1080
GATACATGTG ATCGTCGTCG CAGGGGAGAG TTTTCTTTAT TTCAATCTAG CATGATTGTA 1140
ACAGCTTTAC AATCAAAGTT TGCAGATCCC TATCTTGTAT TTCATGAGCG CTTATCGTCG 1200
AAGTGTCATC GCATAACAGG AACACGTGGC AATCCATCGC TTATATTAAT TCTAGATCGA 1260
CATCCCATAT CCGCTACCGT ATGTTTTCCC ATTGCTCGAC ATTTAACTGG AGATTGTTCC 1320
TTGGAGATGC TAATTAGTAT GATAATAAGG TTGCCCCAGG AACCGCCAGG ATGCAACTTG 1380
GTGATTGTCG ATCTACATGA CGAAAAGGAG CATGTTAGCC GTCTATCTTC ACGGAATAGG 1440
ACCGGCGAGA AAACAGATCT ACTAATGCTC AGGGCACTTA ATGCAGTGTA TTCCTGTTTA 1500
GTAGACACTA TTATGTACGC AAATCATATT TGTCCCTACA GTAAGGATGA ATGGGAATCT 1560
GAATGGTTGG ATCTACCATG GTTTGATACA TCTTTGGCCA CAACGTTTAT AAACGAACCT 1620
CGTACTGATT ATCGCGGTAG TAGGGTGTCA TTACACCATA CGCTTTTAGC GATATTTAAG 1680

CGGCGAGAAT TATGTGCCGA AGATGGTAGC TTATCAACAA CGCATGCATG GATATTGTGG 1740

GGATTATTAA TGAAACTGCG GAACATTAAC GTCGAACGAT TTAATATTAC TGGCCTGTCC 1800

ACAACAAAGT GTGTAGAATC GTTCATGGAT ACTATGTCGG AGAGATTGGT AACACATAGT 1860

AGCTGGAATG ATGCCTTCGA GATTGAAGCT GATGTACTAG CCTATAATAA AGAGATGGCT 1920

ATGTAAAACT ACCCATTCAT ATCGCGCTTC TATAATTAGC TTGCCCACAT CACAATGATG 1980

CGGCAATATT GACTTATATT AAGATAGTAA TTTGGCGTCC TTA 2023

(2) INFORMATION FOR SEQ ID NO:2:

(i) SEQUENCE CHARACTERISTICS:
(A) LENGTH: 2236 base pairs
(B) TYPE: nucleic acid
(C) STRANDEDNESS: single
(D) TOPOLOGY: linear

(ii) MOLECULE TYPE: DNA (genomic)

(iii) HYPOTHETICAL: NO

(iv) ANTI-SENSE: NO

(xi) SEQUENCE DESCRIPTION: SEQ ID NO:2:

TAAATAAAGA ACTTTGGGAA TAACAAGCTA TGTATAGAAT TTATTTCGCG TGAAGATTTT 60

TCCCAAGTCC GATCACATTT CAGGTATTAC AGCGGTAATA GATCCATGCA TTATGAGGGT 120

TTGACGTATT ATCTCGATTA AGAACATATT GTAATACACC CACTGTTTCT CAAACGAGTG 180

TCTATCAATG ATATAATACA TTGATGTATC GACTATAATA CCCCCAATGT TCAAAGGCTG 240

ATAAAACTGA TATATCTATG GCCGCGCATA GCAATTCTGC CGTATCTTCT CCCACCGATT 300

CTCGTAACGC GACGTCTATG GGATCAATGT CTTTATATAG ACCGTCTAGA ATAAGAGCCA 360

GTTTACGTAT CTTGAGGTCC TGTATAGATT TTGGTGCAGA TGTTTCTGCC ACATCCAATA 420

AAGTAGTCTC GTCTGCAAAG GCTGATGGAC TAAGAACTCC ATGTTGCTCT TCCAATGAAG 480

AAGTCCAGTT CACAACTAAT TTCAGTAACC ATGCCAAGAA ATAAAATCCT CTGAATAAAC 540

TGTTTGTTTC TGCAAGACAA GTCGGCATGG AGTAGGCATT CCCCCTCAAT GGTAGAGGTA 600

TGATGATCGC ACAACTCGCG AATTAAGTCA TAACAATTTG GCAGACGATT TAATATATGT 660

ATATACTGAA GCAACAAAAA CTTCTGACTG GGCGATATAT TTTTGTTTTC TGGTCCAACT 720

CCAACAAACA TGGATGCGTG TCTTCCAAAT AAAGCATTTG AAATCATCCC CAACTCACTT 780

TGTATAATTT CCAGGTCGGA TTGAGATCCA TTCTCCGTAT AAGATTAGAA TTTAAATTGA 840

GCATGTTCAT ATTAAAAACC GAGTCTACTT TCCAGAAGAT TTCCCATAAC TTATTTAGAG 900
AAGTAGAGGG TATACAAGAG CTGGTATCGC AACTCCATAT CTTAAATACG GGTGGTATAT 960
ATTTGATTTG TACCAAAGAA CTGAACCGAG CATGTTTTTT CCGTTGTACT GGATTTGTTT 1020
GTTACTGTTC ACGTTCAATT TACCCCGGCT CCAGCCGTCA TATCCCATGC GCGTTGCACA 1080
GTCGTCGTGT TTGCAGCTTT CTTTGCTGTA ACTATAACAT CGACTCGCCT GCCGAATATC 1140
TCTGATGATA ATGCTTCTCT AGGAGTGGGA ATGCCATCAA ATAATCCTTC AACGAGGTCA 1200
CTCAAAGACT TAGGTAATTC AGTCAATCTT GCACAAGTTA GCACAAATGC ATCACGACTG 1260
CACTCATATA CTAAATCTGA ATATATGTCC GTGATTATAG GGAATTCGGG TATATGAATT 1320
GTACGATCAT GTGGAAAATC GTATGCGGCC TGTATCGTTA ACCCAGAAAT TGCATTTGTC 1380
GGTACCATAT ACTTTGCTAT ATCCGGATCA TACGTTTCCA GACAGAGAAG CCCACAAAGC 1440
TCACGTTCAC TGCATATACC ATCACGACTT AACACAGCTA TACTATCGAT GAACAATTCA 1500
TCTTCATCGG AAGAAAAAGC CCACTTCATA CCTCTGCGAA GTAATTCTCG GCGAACATGA 1560
GCTGCCAATG GTTTGGACTG ACCACCACGT AGAACCAACC CAATTTTTGC GAGCTCTGGT 1620
AATACCATCA TCTATACAGC CTGCCTACAG CAAAAAACAA CCGCCGCAAA AAAATACCTT 1680
TATATCCCAT TCCGATACAT AAAACTGGAC ATTCTATAAC GAAAACATGT CCGTATTTAA 1740
TATCCATTGA CTGTCCTCTC TGGACGTAAC CTATATCACT GTAGCGCAAA TCCAATCCTT 1800
GATAACAGCA TTGCGTTAAT CACTGGGTGC ACGGATTAAC GTGTACGTAT TTACTGTCGC 1860
GTCATATGAA CGACAATGAG CTTGGGTATG CAGCTCGTCA TTGAACGCCA TTTGTGGCAA 1920
AGCAATAAGG GTCTCAGACC ATCACATTAT TCGACGAATT GTACTACATA GGCCACCCCT 1980
TGTTTAACTA TGTCAAGCAT GGATTTGGAT ACTATGTCAA CAGAAGCTAA TGAATATACC 2040
ATCCCCCTCA TGAATTGATG ATGGACGATC GGATACATGC GAAAACTCTT GGGTCGTATT 2100
GACCACTATC TGAGGAATTA GATTGGGATG ATATTATGCA CTTTCTCTTA TTTAGGCGAT 2160
ATATTTTACA ATCCAACAGC TATGACATAC ATCCTCAAAT CACCCGTATG TTTACTCTTT 2220
GGCTATCTAC TTTGTC 2236

## Description

### Field of the Invention

The present invention relates to the use of continuous mammalian cell lines for the production of non-recombinant and recombinant Marek's Disease Virus, the production of genetically engineered Marek's Disease Virus and Marek's Disease Virus vectors, continuous mammalian cell lines infected with Marek's Disease Virus, Marek's Disease Virus vectors and vaccines produced using Marek's Disease Virus obtained by such processes and methods of protecting avians against disease resulting from infection by Marek's Disease Virus and/or other pathogens by administration of vaccines produced using such processes. Particularly, the mammalian cell lines capable of infection or transfection with Marek's Disease Virus are feline kidney cell lines.

### Background of the Invention

Marek's Disease (MD) is an acutely oncogenic disease of chickens, which causes lymphomas, visceral tumors, nerve lesions, and immunosuppression. The disease is global and ubiquitous in distribution. The etiologic agent is a herpesvirus, Marek's Disease Virus. Marek's disease has been a primary cause of deaths and condemnations in broiler flocks [Calnek, B.W. and Witter, R.L., Diseases of Poultry, 9th edition, Iowa State University Press, Ames, Iowa, pp 342-385 (1991)].

There are three serotypes of Marek's Disease Virus. Serotype 1 includes all pathogenic strains and their attenuated derivatives. Serotype 2 consists of naturally avirulent chicken viruses, while serotype 3, also known as Herpesvirus of Turkeys (HVT), includes avirulent turkey viruses that are capable of replication in chickens. The three serotypes are partially cross-protective, but can be distinguished using monoclonal antibodies [Silva, R.F. and Lee, L.F., Virol. , vol. 36, pp. 307-320 (1984)] and other known methods.

The first vaccines against MD consisted of live serotype 3 virus. Although effective initially, the emergence of very virulent Marek's Disease Virus (vv Marek's Disease Virus) strains. defined by their ability to break vaccination with HVT, prompted the development of serotype 2 and attenuated serotype 1 vaccines, as well as bivalent and trivalent combinations of serotypes 1, 2 and 3 [Calnek, B.W. and Witter, R.L., Diseases of Poultry, 9th edition, Iowa State University Press, Ames, Iowa, pp. 342-385 (1991)].

Marek's Disease Virus appears to be less recombinogenic than other herpesviruses, and the cell-associated nature of the virus makes plaque purification of recombinants away from parental virus problematic. Nevertheless, recombinant MD viruses have been generated from serotype 1 [Sonoda, K., et al., Vaccine V. 14, pp. 277-284 (1996); Parcells, M.S., et al., J. Virol., V. 69, pp. 7888-7898 (1995); Parcells M.S., et al., Virus Genes, V. 9, pp. 5-13, (1994); Parcells, M.S., et al., J. Virol., V. 68, pp. 8239-8253 (1994); Sakaguchi, M., Vaccine V. 12, pp. 953-957 (1994); Reddy, S.K., et al., Vaccine V. 14, pp. 469-477 (1996)], serotype 2 [Marshall, D.R., et al., Virol. V. 195, pp. 638-648 (1993); Silva, R.F., 14th International Herpesvirus Workshop (Abstract) (1989)]; and serotype 3 [Reddy, S.K., et al., Vaccine V. 14. pp. 469-477 (1996); PCT Patent Application WO 95/29248 (1995); Silva, R.F., 14th International Herpesvirus Workshop (Abstract) (1989); Darteil, R., et al., Virol. V. 211, pp. 481-490 (1995): U.S. Patent No. 5,187,087 issued in 1995; Zelnik, V., et al., J. Gen. Virol. V. 76, pp. 2903-2907 (1995); PCT Patent Application WO 93/25665, published (1993); Ross, L. J. N., et al., J. Gen. Virol., V. 74, pp. 371-377 (1993); Sondermeijer, P.J.A., et al., Vaccine V. 11, pp.349-358(1993); European Patent No.431,668 B1, published (1995); Morgan, R.W., et al., Avian Dis., V. 36, pp. 858-870 (1992); Bandyopadhyay, P.K., et al., 13th International Herpesvirus Workshop 323 (Abstract) (1988)]. In all of the above mentioned references, the viruses were replication competent and were produced in primary avian cells.

Commercially available Marek's Disease Virus vaccines, with the exception of some monovalent HVT formulations, consist largely of live Marek's Disease Virus-infected primary chicken embryo fibroblast (CEF) cells. A significant problem associated with using whole live Marek's Disease Virus-infected primary chicken cells to grow Marek's Disease Virus for use in vaccines is that the CEF cells must be stored at liquid nitrogen temperatures and administered by injection in order to be effective. Whole live cell vaccines have been previously necessary since the three Marek's Disease Virus serotypes are strongly cell-associated in cell culture and in most tissues of an infected bird. Spread of infection within birds can be achieved by direct cell to cell contact, with little or no cell-free virus being released. Infectious virions are produced only in the feather follicle epithelium (FFE), and are responsible for bird-to-bird transmission [Calnek, B.W., et al., Avian. Dis., V. 14, pp. 219-233 (1970); Witter, R.L., et al., J. Natl. Cancer Inst., V. 49, pp. 1121-1130 (1972); Eidson, C.S., et al., J. Natl. Cancer Inst., V. 47, pp. 113-120 (1971)].

Commercial cell-free Marek's Disease Virus vaccines can be made by cell culture. However, the production of cell-free Marek's Disease Virus vaccines has been thus far been limited to vaccines produced using only serotype 3 Marek's Disease Virus. This is because only serotype 3 Marek's Disease Virus makes free virions in sufficient quantities for production of Marek's Disease Virus vaccines. It has been suggested that lack of expression of the viral glycoprotein D (gD) gene may be involved in limiting release of cell-free virions [PCT Patent Application WO 95/29248 published (1995); Tan, X. and Velicer, L.F., 18th International Herpesvirus Workshop A, 145 (Abstract) (1993)].

In addition to CEF, other primary avian cells have been used to grow Marek's Disease Virus, including chicken kidney (CK) and duck embryo fibroblast (DEF). Only two continuous avian cell lines have been described as being capable of growing certain Marek's Disease Virus serotypes. Serotype 3 Marek's Disease Virus (Marek's Disease Virus-3) has been described as growing on a chemically transformed quail cell line designated QT35 [Niikura, M., Narita, T. et al.. J. Vet. Med. Sci., V. 53, pp. 439-446 (1991)]. A chemically transformed CEF cell line designated CHCC-OU2 [Ogura, H. and Fujiwara, T., Acta Med. Okayama, V. 41, pp. 141-143 (1987)] has been described as supporting the growth of Marek's Disease Virus-1 [Abujoub, A. and Coussens, P.M., Virol., V. 214, pp. 541-549 (1985)].

Other known processes for producing Marek's Disease Virus include the use of tumorogenic or oncogenic cell lines. Marek's Disease Virus-transformed lymphoblastoid cell lines [Nazerian, K. Avian Pathol. V. 16, pp. 527-544 (1987)] are derived from lymphoid tumors in chickens infected with oncogenic Marek's Disease Virus-1. The viral genome is maintained in a latent or semi-latent state in these cells, such that transmission of infection to co-cultivated CEF cells or DEF cells occurs at a low frequency, if at all. In addition, these lymphoblastoid lines are refractory to superinfection with other Marek's Disease Virus viruses. Furthermore, Marek's Disease Virus-transformed lymphoblastoid cell lines have not demonstrated utility in the production of nonrecombinant (conventional) Marek's Disease Virus vaccines or in the preparation of recombinant Marek's Disease Virus viruses or genetically altered Marek's Disease Virus viruses or vectors.

Similarly, lymphoblastoid cell lines [Nazerian, K., Avian Pathol., V. 16, pp. 527-544 (1987)] derived from oncogenic avian retroviruses (avian leukosis virus and reticuloendotheliosis virus) are not useful for the production of commercial Marek's Disease Virus vaccines or for the generation of recombinant Marek's Disease Virus, due to the shedding of retroviruses, the poor growth characteristics of lymphoblastoid cells, and the low level of productive Marek's Disease Virus infection.

Mammalian primary cells and mammalian cell lines have never been described as suitable substrates for the growth of any serotype of Marek's Disease Virus, nor has any mammal ever been demonstrated to be productively infected with Marek's Disease Virus.

### Brief Description of the Drawings

FIG. 1 describes the insertion of the Marek's Disease Virus-1 gH gene into the map of the pCR 3.1 vector.

FIG. 2 is a map of the pGreen Lantem2 plasmid.

FIG 3. describes the reverse transcriptase PCR analysis of Marek's Disease Virus gH transfected NLFK 3 G4 cells.

### Summary of the Invention

In one aspect this invention relates to a method for producing MDV which comprises:
(a) infecting or transfecting a continuous mammalian cell line with MDV; and
(b) culturing the continuous mammalian cell line infected or transfected with MDV. The MDV can be a non-recombinant molecule comprising the nucleic acid sequence of Marek's Disease virus or a fragment(s) thereof, a recombinant molecule comprising the nucleic acid sequence of Marek's Disease Virus or a fragment(s) thereof, a recombinant molecule comprising the nucleic acid sequence of MDV and at least one heterologous gene or fragment(s) thereof inserted into the nucleic acid sequence of MDV, and a recombinant molecule comprising the nucleic acid sequence of MDV from which one or more genes, regulatory genetic elements, or fragment(s) thereof has been deleted. For example, the deleted gene can be one for essential for replication. More particularly, the deleted gene can be the gH gene of Marek's Disease Virus or a fragment(s) thereof.

The MDV can be selected from the group consisting of serotype 1, serotype 2 and serotype 3, taken singly, or in any combination thereof.

In another aspect the invention relates to the above described method wherein the MDV is a virus used for the preparation of a vaccine capable of inducing protection against disease in avians.

Preferably, the continuous mammalian cell line used in the above mentioned method is a feline kidney cell line infected with the Md5 strain of MDV. such as cell line ATCC No ATCC-CRL12336. Examples of other suitable strains include the MDV-1 strains 584A and 652.

Yet another aspect of the invention is a continuous mammalian cell line infected or transfected with MDV. The MDV can be a non-recombinant molecule comprising the nucleic acid sequence of Marek's Disease Virus, or a fragment(s) thereof, a recombinant molecule comprising the nucleic acid sequence of MDV or a fragment(s) thereof. a recombinant molecule comprising the nucleic acid sequence of MDV and at least one heterologous gene or fragment(s) thereof inserted into the nucleic acid sequence of MDV and a recombinant molecule comprising the nucleic acid of MDV from which one or more genes, regulatory genetic elements, or fragment(s) thereof has been deleted. For example, the cell line can be one wherein the deleted gene is essential for replication. More particularly, the cell line can be one wherein the deleted gene is gH or a fragment(s) thereof.

Preferably, the continuous mammalian cell line is a feline kidney cell line infected with the Md5 strain of MDV, such as cell line ATCC No ATCC-CRL12336. Examples of other suitable strains include the MDV-1 strains 584A and 652.

The MDV can be selected from the group consisting of serotype 1, serotype 2 and serotype 3, taken either singly or in any combination thereof.

In a further aspect of the invention, the MDV is a virus used for the preparation of a vaccine capable of inducing protection against disease in avians.

Yet another aspect of the invention is a method for producing a MDV vaccine comprising culturing a continuous mammalian cell line infected or transefected with MDV and harvesting cell culture components therefrom.

Still another aspect of the invention is a method for producing a continuous mammalian cell line infected with MDV which comprises:
(a) culturing continuous mammalian cells with MDV; and
(b) propagating the MDV infected or transfected mammalian cells to produce the cell line.

Another aspect of the invention is a vaccine comprising MDV produced by the above mentioned method. The MDV can be a non-recombinant molecule comprising the nucleic acid sequence of Marek's Disease Virus, or a fragment(s) thereof. a recombinant molecule comprising the nucleic acid sequence of MDV, or fragment(s) thereof. a recombinant molecule comprising the nucleic acid sequence of MDV and at least one heterologous gene or fragment(s) thereof inserted into the nucleic acid sequence of MDV, a recombinant molecule comprising the nucleic acid sequence of MDV from which one or more genes, regulatory genetic elements, or fragment(s) thereof, has been deleted. For example, the MDV can be one wherein the deleted gene is essential for replication. More particularly, the MDV can be one wherein the deleted gene is gH or a fragment(s) thereof.

Preferably, the vaccine is prepared using a continuous mammalian cell line which is a feline kidney cell line infected with the Md5 strain of MDV. such as cell line ATCC No ATCC-CRL12336. Examples of other suitable strains include the MDV-1 strains 584A and 652.

The vaccine can be comprised of MDV selected from the group consisting of serotype 1, serotype 2 and serotype 3, taken either singly or in any combination thereof. Preferably the vaccine is produced using a feline kidney cell line infected or transfected with Marek's Disease Virus.

Still another aspect of the invention is a recombinant MDV produced using the above mentioned method. The recombinant MDV can, for example, comprise the nucleic acid sequence of naturally occuring MDV from which one or more genes has been deleted, such as a gene essential for viral replication. An example of such as essential gene is the glycoprotein H (gH) gene, or fragment thereof, of the MDV genome. The resulting disabled (replication defective) virus is infectious for a single cycle provided that a complementary cell line, genetically engineered to contain the gene essential for viral replication and thereby the expression product of the deleted gene, is available to propagate the disabled virus. The production of disabled viruses and their use as vaccines is described in PCT Application GB91/01632 (Publication No. WO 92/05263), which is incorporated herein in its entirety.

Yet another aspect of the present invention is a cell line which comprises a genetically engineered mammalian cell line, which is capable of expressing a gene of naturally occuring MDV which is essential for replication, such as for example, gH, and which is capable of replication of a disabled MDV virus.

Yet another aspect of the invention is a MDV vector produced using the method described above. The MDV vector comprises recombinant MDV and one or more heterologous genes or fragment(s) thereof. Optionally, the MDV vector has a 3' flanking region comprising a nucleic acid sequence as described in SEQ ID NO: 2. Also optionally, the MDV vector can have a 5' flanking region comprising a nucleic acid sequence as described in SEQ ID NO: 1. Furthermore, the MDV vector can have both a 3' flanking region comprising a nucleic acid sequence as described in SEQ ID NO: 2 and a 5' flanking region comprising a nucleic acid sequence as described in SEQ ID NO: 1.

Yet another aspect of the invention relates to a method for protecting animals against disease by admininstering to such animals a vaccine comprising MDV produced by the method described above. Preferably, the the animal is avian.

### Detailed Description of the Invention

The present invention provides processes for the use of continuous mammalian cell lines for the production of non-recombinant and recombinant Marek's Disease Virus (MDV), the production of genetically engineered MDV and MDV vectors, continuous mammalian cell lines infected or transfected with MDV and vaccines capable of protecting animals against disease by MDV and/or other disease causing agents produced using such processes. Also provided are methods of administering MDV vaccines and MDV vector-produced vaccines to animals for protection against infection by MDV.

By "MDV" is meant a nucleic acid sequence encoding for the genome of Marek's Disease Virus or any fragment or fragments (fragment(s)) thereof. MDV includes, for example, the genome of naturally occurring Marek's Disease Virus (non-recombinant Marek's Disease Virus) or any fragment thereof, recombinantly produced Marek's Disease Virus (recombinant Marek's Disease Virus) or any fragment thereof, genetically engineered or altered Marek's Disease Virus or any fragment(s) thereof. Fragment(s) of the nucleic acid sequence of non-recombinant Marek's Disease Virus, recombinant Marek's Disease Virus or genetically engineered or altered Marek's Disease Virus can include one or more genes of Marek's Disease Virus or parts thereof. Preferably, such fragment(s) encode for antigens of Marek's Disease Virus which are useful as vaccines against Marek's Disease Virus.

The MDV can be of serotypes 1, 2 or 3 taken singly or in any combination thereof. The MDV can further be of any combination of strains of serotypes 1, 2 or 3 taken singly or in any combination thereof.

It is understood that MDV includes, for example. substitutions, insertions, inversions, additions and deletions to the nucleic acid sequence of Marek's Disease Virus, and any combinations thereof, such as deletion mutants of Marek's Disease Virus and Marek's Disease Virus from which a non-essential region, such as a non-essential gene, of the genome of Marek's Disease Virus, or altematively, an essential region of the genome of Marek's Disease Virus is missing. Such variations can be naturally occurring or can be genetically engineered using conventional recombinant techniques to bring about such variations. Production and manipulation of recombinant Marek's Disease Virus or fragments thereof, genetically engineered or altered Marek's Disease Virus or fragment(s) thereof and recombinantly produced variations of naturally occurring Marek's Disease Virus are within the skill in the art and can be carried out according to recombinant techniques described, among other places, in Maniatis, *et al.*, 1989, Molecular Cloning, A Laboratory Manual, Cold Spring Harbor Laboratory Press, Cold Spring Harbor, NY; Ausubel, et al., 1989, Current Protocols In Molecular Biology, Greene Publishing Associates & Wiley Interscience, NY; Sambrook, *et al.*, 1989, Molecular Cloning: A Laboratory Manual, 2d ed., Cold Spring Harbor Laboratory Press, Cold Spring Harbor, NY; Innis *et al.* (eds), 1995, PCR Strategies, Academic Press, Inc., San Diego; and Erlich (ed), 1992, PCR Technology, Oxford University Press, New York, all of which are incorporated herein by reference.

The preparation of various recombinant Marek's Disease Virus and Marek's Disease Virus vectors using recombinant techniques is known. For example, US Patent No. 5,231,023 issued July 27, 1993 describes a Marek's Disease Virus viral vector prepared by insertion of a heterologous gene into a nonessential region of the DNA genome of Marek's Disease Virus.

An example of MDV from which an essential gene is missing is a replication-defective Marek's Disease Virus. A particular example of a replication defective MDV is the genome of Marek's Disease Virus from which the gH gene has been deleted using recombinant techniques.

It is understood that MDV also includes recombinant Marek's Disease Virus comprising a heterologous gene or genes incorporated into an insertion region located within the nucleic acid sequence of the genome of Marek's Disease Virus. Preferably, the heterologous gene or genes encode for antigens of other disease causing agents, such as, for example, avian disease causing agents. Methods for preparing recombinant Marek's Disease Virus containing heterologous genes are within the skill in the art and can be carried out according to recombinant techniques described in the Maniatis, *et al.*, and other references noted above.

By "infection" or "tranfection" is meant the DNA transfer of virus or fragment(s) thereof into mammalian cells. Methods for gene transfer into mammalian cells is within the skill in the art as described, for example, by Watson et al., Recombinant DNA, 2nd edition, W. H. Freeman and Company, N.Y., (1992).

By "MDV vector" is meant an expression system containing appropriate regulatory sequences necessary for expression of one or more functional polypeptides, such as antigens, encoded by the vector, which contains MDV (such as the genome of non-recombinant Marek's Disease Virus or any fragment thereof, recombinant Marek's Disease Virus or any fragment thereof, or genetically engineered or altered Marek's Disease Virus or any fragment(s) thereof), and which contains one or more heterologous genes (genes other than those naturally associated with Marek's Disease Virus) which are capable of being expressed in the continuous mammalian cell lines of the present invention or produced using the processes of the present invention. The MDV vector can, for example, contain the nucleic acid sequence of recombinant Marek's Disease Virus and one or more heterologous genes which encode antigens from other disease causing agents, such as for example, avian disease causing agents. The heterologous gene(s), which can be inserted into the MDV vector using recombinant techniques within the skill of those in the art, can be expressed in the continuous mammalian cell lines of the present invention and. once given to animals, such as avians, elicit an immune response to both recombinant Marek's Disease Virus and the heterologous gene expression products or polypeptides, such as antigens. An adequate and functional promoter should be linked to the heterologous gene(s) so that the MDV vector is capable of expressing the heterologous gene(s). The promoter can be any eucaryotic, procaryotic, or viral promoter capable of directing transcription in cells infected with the recombinant MDV vector. The preparation and use of promoters is known and can be carried out according to recombinant techniques described in the Maniatis, *et al.*, and other references noted above.

Alternatively, the MDV vector can be missing a Marek's Disease Virus gene necessary for replication, that is, it can be replication-defective and contain also one or more heterologous genes which encode antigens from other disease causing agents, such as avian disease causing agents. One can prepare such recombinant MDV vectors using known recombinant techniques described in the Maniatis, *et al.*, and other references noted above.

It is understood the present invention provides processes for producing multivalent vaccines for protecting animals against infection by disease causing agents, i.e. in addition to Marek's Disease Virus.

By "continuous cell line" is meant immortalized cells which can be maintained *in* vitro for at least 50 cell passages, preferably between 15 and 20 passages.

The present invention encompasses processes for producing MDV and MDV vectors by infecting and culturing continuous mammalian cell lines and cell lines which are subcloned from, or otherwise derived from such continuous mammalian cell lines, such as for example. feline kidney cell lines, which are capable of producing MDV or useful in the production of MDV vectors. The MDV produced in such cell lines can then be isolated using isolation techniques known to those of skill in the art.

The present invention also encompasses the continuous mammalian cell lines infected with MDV or a MDV vector. The mammalian cell line for infection or transfection with MDV is characterized as a continuous cell line free of mammalian and avian viruses. Preferably, the mammalian cell line is a kidney cell line. Kidney cell lines can be cultured and maintained using known mammalian cell culture techniques such as described in Celis, Julio, ed.. Cell Biology, Laboratory Handbook, Academic Press, N.Y. (1994). Varying culturing conditions for mammalian cells, including media formulations with regard to specific nutrients, oxygen tension, CO₂ and reduced serum, can be selected and optimized by one of skill in the art.

The preferred mammalian kidney cell line for infection or transfection with MDV is a feline kidney cell line. Any feline kidney cell lines can be used to practice the processes of the present invention, such as for example, feline kidney cell lines designated as CRFK, deposited with the American Type Culture Collection, 12301 Parklawn Drive. Rockville, Maryland and designated ATCC CCL-94. A preferred cell line is a feline kidney cell line and any clones thereof, designated as the Norden Laboratories Feline Kidney (NLFK) cell line deposited on April 8, 1997. with the American Type Culture Collection, and designated as ATCC CRL-12336. The depositor was Pfizer Inc, Central Research Division, Eastern Point Road, Groton, CT 06340, USA. The ATCC CRL-12336 cell line is a feline kidney cell line infected with the Md5 strain of Marek's Disease Virus. The Md5 strain was obtained from the American Type Culture Collection having the designation ATCC VR 987.

Mammalian cell lines, preferably feline kidney cell lines for use in the present invention can be cloned using known mammalian cell culture techniques familiar to one skilled in the art. The cells can be cultured and expanded from a single cell using several commercially available culture media under known conditions suitable for propagating mammalian cells.

For example, feline kidney cells, such as the NLFK cells, kept frozen until use, can be warmed to a temperature of about 37° to about 38.5 °C and added to a suitable growth medium such as OptiMEM (Life Technologies Inc.) containing 2% fetal bovine serum (FBS). The cells can be incubated at a temperature of about 37° to about 38.5 °C in a humidified incubator with about 5% CO₂ until confluent. In order to passage the cells, the growth medium can be removed and 0.05% trypsin and 0.53mM EDTA added to the cells. The cells will detach and the cell suspension can be collected into centrifuge tubes and centrifuged to cell pellets. The trypsin solution can be removed and the cell pellet resuspended into new growth medium. The cells can then be further propagated in additional growth vessels to a desired density.

Infection or transfection can occur by co-cultivation with MDV-infected cells where the MDV is non-recombinant, recombinant, genetically engineered or is an MDV vector. The cells used for co-cultivation can be MDV-infected cells such as CEF cells or DEF cells, lymphoblastoid cells, peripheral blood mononuclear cells, CHCC-OU2 cells, QT35 cells, and clones of feline kidney cells such as the 3G4 clone described in the Examples. Alternatively, mammalian cell lines may be infected with MDV by introduction of purified MDV DNA or DNA from MDV-infected cells into the mammalian cell lines using methods which are known to those of sill in the art, such as calcium phosphate coprecipitation, DEAE dextran, polybrene, lipofection, and electroporation. Preferably, DEF or CEF MDV-infected cells are used to infect the continuous mammalian cell line of the present invention. The cultivation of MDV-infected CEF, DEF, lymphoblastoid, peripheral blood mononuclear cells, CHCC-OU2 cells, and QT35 cells are known to those of skill in the art.

For example, feline kidney cells can be infected with MDV by growing the cells to about 50 to about 80% confluency, preferably 80% confluency and adding DEF cells infected with MDV to the semi-confluent cells. The cells can be incubated at a temperature of about 37 to about 38.5°C for about 48 hours. The growth medium can then be removed and replaced with fresh medium. The cells can then be incubated for about 48 to 72 hours, preferably 48 hours, at a temperature of about 37 to about 38.5°C. The medium can then be removed and replaced with medium containing 2% FBS and the cells incubated for at least 24 hours. The cells can then be split by trypsinization as described above. Infected cells can be monitored by indirect fluorescent antibody (IFA) using an MDV-specific antibody. MDV-specific antibody can be produced using known monoclonal hybridoma techniques [Goding, James, W., Monoclonal Antibodies: Principles and Practice, second edition, Academic Press (1986); Harlow, E., et al., Antibodies, a Laboratory Manual, Cold Spring Harbor Laboratory (1988)]. A frozen stock of the MDV-infected cells can then be stored until ready for use.

The present invention also relates to processes for production of vaccines comprising the MDV produced by infecting continuous mammalian cell lines, such as for example, feline kidney cell lines, and culturing the infected cell lines to produce antigens useful as vaccines against MDV. The MDV infected cells can be used as vaccines against MDV or the cell-free MDV isolated from the cells can be used as vaccines.

The present invention further relates to processes for producing vaccines comprising the MDV produced by transfecting or infecting continuous mammalian cell lines, such as feline kidney cell lines, with an MDV vector comprising MDV and the genes of one or more heterologous proteins or polypeptides, which is capable of expressing heterologous genes in addition to MDV, which are useful against avian disease causing agents other than MDV.

Furthermore, the invention relates to processes for producing vaccines produced by transfecting continuous mammalian cell lines with an MDV vector comprising replication-defective MDV and the genes or parts thereof of one or more heterologous proteins or polypeptides, which is capable of expressing heterologous genes in addition to MDV, which are useful against avian disease causing agents other than MDV.

Examples of other avian disease causing agents which may be useful for the production of vaccines and which are produced using the processes of the present invention include Newcastle Disease Virus (NDV), Infectious Bursal Disease Virus (IBDV), Infectious Bronchitis Virus (IBV), Chicken Anemia Virus (CAV), Infectious Laryngotracheitis Virus (ILTV), Avian Leukosis Virus (ALV), Reticuloendotheliosis Virus (REV) and Avian Influenza Virus (AIV).

MDV produced using the process of the present invention containing and expressing one or more different heterologous proteins or polypeptides can serve as a monovalent or multivalent vaccine. Such vaccines can be prepared by methods well known to those skilled in the art of preparation of vaccines.

The present invention further comprises methods of administering MDV vaccines and MDV vector-produced vaccines to animals for protection against infection by MDV. The MDV expressing MDV and/or one or more heterologous proteins or polypeptides of avian disease causing agents such as pathogens can be used to vaccinate animals, such as chickens and turkeys, susceptible to such disease causing agents. Vaccination with the MDV or other antigens produced using the processes of the present invention results in a protective immune response so that the inoculated animals will be protected from subsequent infection by those disease causing agents.

The following examples further illustrate but do not limit the present invention.

### Example 1

### Propagation Of Uninfected Continuous Feline Kidney Cell Line

Fourteen cloned cell lines were derived from parent NLFK cells by end point dilution in 96- well tissue culture plates. The cells were expanded in wells derived from a single cell. Following several passages, cells from each clone were frozen and stored at -70°C. All feline kidney cells were propagated in OptiMEM supplemented with 3% fetal bovine serum (FBS) and 22.5 µg/ml gentamicin. Monolayers of NLFK cells were maintained on OptiMEM with varying concentrations of FBS as described in the Examples below.

### Example 2

### Infection Of Monolayers And Freshly Planted Cultures Of NLFK Cells

Cultures of DEF or uncloned NLFK cells in 24 well tissue culture plates were inoculated with the Md5 strain of MDV serotype 1. Preparation and propagation of DEF cells were performed as described by Solomon. J.J., Tissue Culture Association, vol. 1, pp. 7-11 (1975). Embryonated duck eggs were obtained from Comell University.

Virus was detected with an indirect fluorescent antibody (IFA) assay using MDV-specific monoclonal antibodies [Silva, R.F. and Lee, L. F., Virol., vol. 36, pp. 307-320 (1984)] to glycoprotein B (1AN86) or to phosphoprotein 38 (H19). These antibodies were a generous gift of Dr. Lucy Lee of the USDA's Avian Disease and Oncology Laboratory (East Lansing, Ml). Briefly, cells to be examined were fixed with 80% acetone, incubated with one or both monoclonal antibodies, washed and incubated with an anti-mouse FITC conjugate (Kirkegaard & Perry Laboratories). Stained cell preparations were examined with a UV microscope and positive cells were indicated by characteristic FITC fluorescence.

For positive control, an MDV seed consisted of DEF infected cells in which approximately half of the cells were expressing MDV antigen when stained by IFA. The seed was diluted to an estimated 100 IFA positive cells/well. Duplicate wells were stained periodically by IFA for the presence of MDV infection. Not all wells containing NLFK cells demonstrated the presence of replicating MDV. Only 3 out of a total of 48 wells demonstrated growth of MDV by IFA. The dilution of MDV infected DEF inoculum used in this experiments resulted in evenly distributed single cells on the bottom of a 24 well plate containing no preexisting cell monolayer. Therefore, the presence of the multicell fluorescent patches indicated replication of the virus in the feline cells.

A study was conducted to determine if MDV replicated better in freshly planted cells or cells in a confluent monolayer. Monolayer cultures had been planted 4 days prior to infection, while freshly planted cells had been seeded approximately 4 hours prior to infection. The MDV infected DEF inoculum contained approximately 3000 IFA positive cells/well in a 6-well dish. As a control, inoculum only was added to several wells. The presence of MDV antigen in cells was periodically monitored by IFA staining. At 5 days following inoculation, 12 wells were trypsinized and used to plant a 75 cm² flask and additional 24 well plates. Five days later the 75 cm² flasks and half of the wells in the 24 well plates were refed with fresh medium. The presence of MDV antigen in the 24 well plates was monitored by IFA staining at 4 days and 12 days following the initial passage. Cells from the 75cm² flask were passaged to 24 well plates 2 days following the medium change (7 days post-passage). These cells were monitored by IFA staining five days later.

There was little difference in the ability of MDV to replicate on monolayers of NLFK cells or on freshly planted cells. Further, this experiment demonstrated that in the absence of NLFK cells, the MDV infection of DEF cells diminished and was lost by the third passage. In the presence of NLFK cells, the MDV infection is maintained and can be enhanced during passage of the infected cells. This study supports the suggestion that replication of MDV in NLFK cells is enhanced by a medium change during the infection. Both the number and the size of fluorescent foci was increased when the medium was changed at 5 days following passage.

### Example 3

### Infection Of Feline Kidney Cells Cloned From NLFK Cell Line

Fifteen clones of feline cells derived from the NLFK cell line were evaluated for their ability to support growth of the Md5 isolate of MDV. The clones were grown in 75 cm² flasks. One ml of a suspension of MDV infected DEF cells containing approximately 5000 positive IFA cells was added to each flask. As controls, flasks containing the parental NLFK line or no cells were also inoculated. After 7 days, the flasks were passaged into additional 75 cm² flasks and 24 well plates. The 24 well plates were periodically monitored by IFA staining for the presence of MDV antigens in the NLFK cells. After 6 days, the flasks were refed with fresh medium. From the results of the IFA stains, the four clones showing the most aggressive MDV replication were chosen to be passaged to the next level. Eight clones showed little or no MDV spread, while the remaining three cell clones gave intermediate results. Two days after the medium change these flasks were trypsinized and used to seed two 75 cm² flasks and additional 24 well plates, which were periodically monitored by IFA staining for the presence of MDV.

These results suggest that the reason uncloned NLFK cells initially demonstrated only a few positive wells is that a subpopulation of NLFK cells are capable of supporting MDV replication. Although several clones appeared to be able to support the growth of MDV to some extent, four clones demonstrated good MDV replication under the conditions used in these experiments. These were clones 3G4, 9, 7 and KKCL6. Clones 3G4 and 9 were the best of the four and were selected to be passaged further. It should also be noted that the DEF cell inoculum lost the MDV infection by the first passage. As noted before, medium change during incubation enhanced the spread of MDV infection.

### Example 4

### Preparation of MDV-Infected Feline Kidney Cell Stocks

After several additional passages, cultures of NLFK clones 3G4 and 9 were obtained in which nearly all cells express the gB and pp38 antigens as determined by IFA. These cultures do not show the typical MDV-induced CPE seen in infected DEF cultures.

Frozen cell stocks were prepared from passages 5 and 6, and these were used as seed stock for the *in vivo* experiment described below.

### Example 5

### Inoculation Of Chickens With NLFK MDV-Infected Cells and MDV Virus

*In vivo* experiments were performed using specific pathogen-free chickens (strain RF1-5) obtained from Hy-Vac. The feline cell line used in this work was obtained from Pfizer Animal Heath, Lincoln as Norden Laboratories Feline Kidney (NLFK) cells. Fifteen cloned cell lines were derived from the original NLFK line by end point dilution in 96 well tissue culture plates. Two of the NLFK cell clones, designated clone 3G4 and clone 9, were used in the studies. CHCC-OU2 cells [Ogura, H. and Fujiwara, T., Acta Med., Okayama. vol. 41, pp. 141-143, (1987)] were provided by Dr. Douglas N. Foster (University of Minnesota). The very virulent MDV strain Md5 [Witter, R. L. *et al.*, Avain Res., vol. 24, pp. 210-232, (1980)] was purchased from the American Type Culture Collection (ATCC VR-987) and was used in this study at cell culture passage level 11.

Six groups of 10 chicks each at 3 days of age were inoculated intraperitonealy with (1) a mixture of uninfected NLFK clone 3G4 and NLFK clone 9 cells (cell control), (2) Md5-infected DEF cells (positive control), (3) Md5-infected OU2 cells, (4) Md5-infected NLFK clone 3G4 cells, (5) Md5-infected NLFK clone 9 cells. The remaining group (6) was an uninoculated negative control. The birds were inoculated with between 500 and 2000 TCID₅₀ of MDV per chick . Titration of virus was determined using the TCID₅₀ method on monolayers of secondary DEF cells fixed with 80% acetone 6-8 days following infection and examined by IFA as described above.

Infected and uninfected cells were grown at 38.5°C and 5% CO₂ in humidified incubators in OptiMEM (Life Technologies) supplemented with gentamicin and appropriate amounts of fetal bovine serum (0-5%).

Birds were necropsied if they died during the study and examined for signs of MDV infection. Kidney, spleen, liver and brain were harvested for histological evaluation from surviving birds. Blood was collected at day 14 post inoculation for virus isolation from peripheral blood lymphocytes (buffy coats) and plated on DEF cell monolayers.
All chickens injected with Md5-infected cells (groups 2-5) developed classical signs of very virulent MDV, and the majority of these birds died of MD. The negative control and cell control groups did not develop signs of MD for the duration of the experiment (8 weeks of age). The results are shown in Table 1.

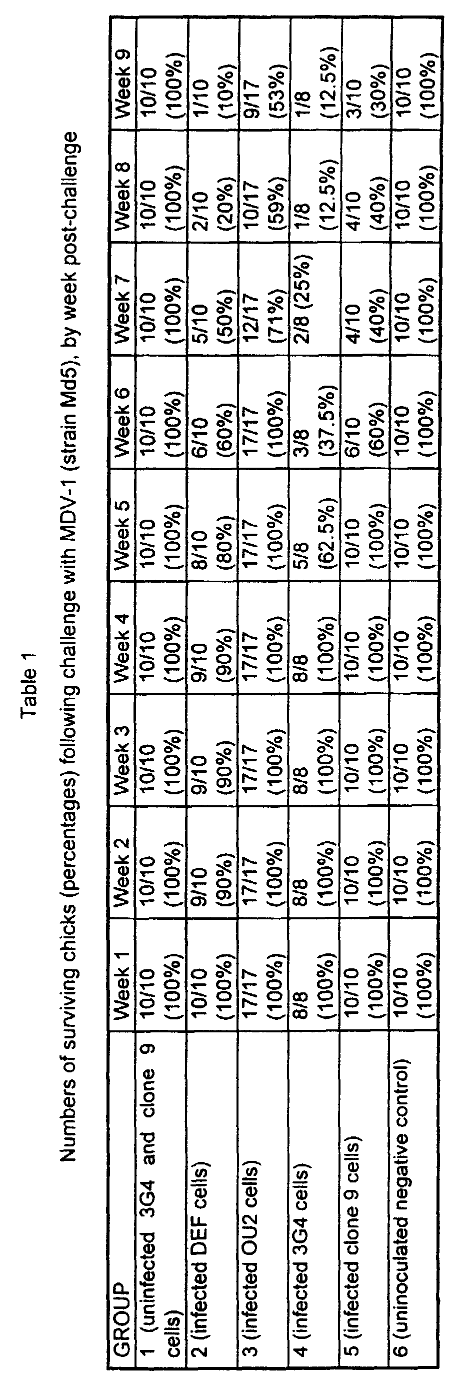

Histopathological evaluation of tissues (kidney, spleen, liver, heart and brain) revealed lesions which were consistent with Marek's disease in surviving birds from the infected groups. No such lesions were seen in birds from the cell control or negative control groups.

For virus reisolation, between 0.5 and 2ml of chicken blood was collected in heparinized tubes by wing vein puncture from each chick on day 14 post-inoculation. The blood from each experimental group was pooled and centrifuged. The buffy coat cells were removed from each tube and used to inoculate DEF monolayers. At day 4 post inoculation the cells were fixed with 80% acetone and prepared for IFA. The cell control and negative control groups were negative by IFA for MDV. The positive control, OU2, NLFK clone 3G4, and NLFK clone 9 groups were positive for MDV by IFA.

### Example 6

Two additional isolates of MDV-1, designated 652 and 584A, were adapted to growth in the 3G4 clone of NLFK cells. These isolates are more recent and more virulent then the Md5 isolate [Witter, R.L., Avian Dis., V. 41, pp. 149-163 (1997)]. The methods used to adapt these isolates to growth in 3G4 cells are essentially identical to those described in Example 3 for adaptation of isolate Md5 to growth in various clones of NLFK cells.

### Example 7

### Preparation of NLFK 3G4 MDV gH Expressing Cell Line

### Preparation of PCR3.1gH Construct

The 2.6kb Glycoprotein H (gH) gene from Marek's Disease Virus strain MD5 was isolated by polymerase chain reaction (PCR) and cloned into pCR2 TA cloning (TA cloning vector, available from Invitrogen Inc., U.S.A., catalogue number K2000-1) by polymerase chain reaction (PCR) using a commercially available PCR regent system (GIBCO-Life Technologies, cat. No. 10198-018), using an upper PCR primer 5'-GGGGG TACCA AGTTG CATTG GATGG CTACA TA-3' and a lower PCR primer 5'-GGGGC TAGCT TAAAG ATCGT CGTAC AGGCT CAA-3'. The gH gene can be sequenced using known techniques. The sequence for the gH gene is described by Scott et al., J. Gen. Virol. vol. 74, pp. 1185-1190, (19930.

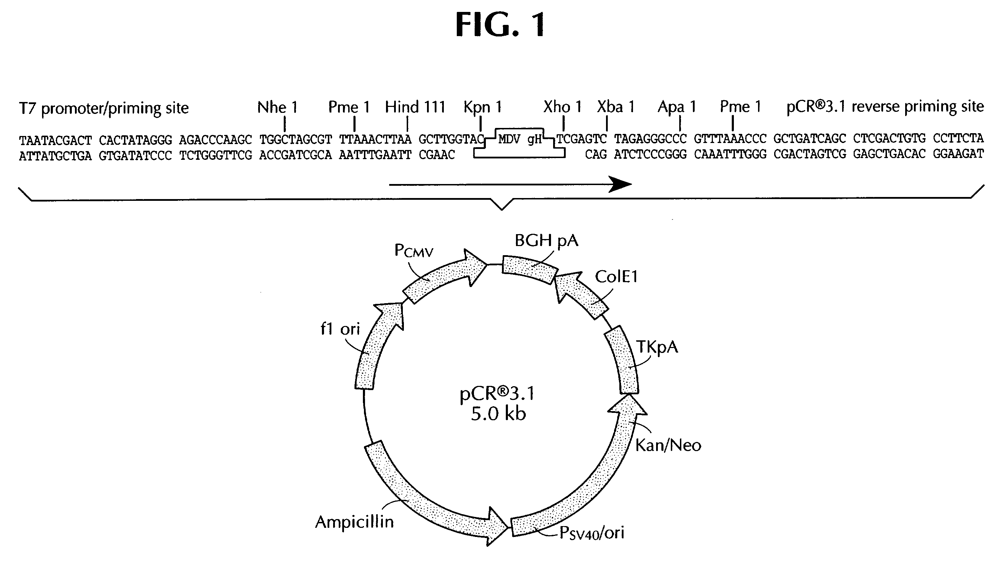

The MDV gH gene was then subcloned into pCR3.1 vector (available from Invitrogen Inc., U.S.A., Catalog Number K3000-01) utilizing the Kpnl and Xhol sites in the multiple cloning site (see Figure 1). The vector pCR3.1 is a eukaryotic expression vector containing cytomegalovirus (CMV) immediate-early promoter for efficient mammalian expression and the neomycin resistance gene to allow positive selection for cells that contain the vector. The vector pCR 3.1 is characterized by the following: CMV promoter bases 1-596; putative transcriptional start bases 520-625; T7 promoter/priming site bases 638-657; multiple cloning site bases 670-785; pCR 3.1 reverse priming site bases 797-815; BGH polyadenylation site bases 796-1010; ColE1 oring bases 1100-1773; SV40 promoter and origin bases 3178-3515; Neomycin/Kanamycin resistance gene (ORF) bases 2355-3143; Thymidine kinase polyadenylation site bases 1910-2180; ampicillin resistance gene bases (ORF) bases 3595-4455; and f1 origin bases 4586-5042. The construct was confirmed by sequence analysis and restriction analysis using known techniques. Using an *in vitro* transcription/translation system (available from Promega, U.S.A., TnT T7 System Catalog Number L1170), the construct was able to express a protein of the expected size of approximately 90 kDa.

### Generation of NLFK 3G4 MDV gH Stable Transfectants

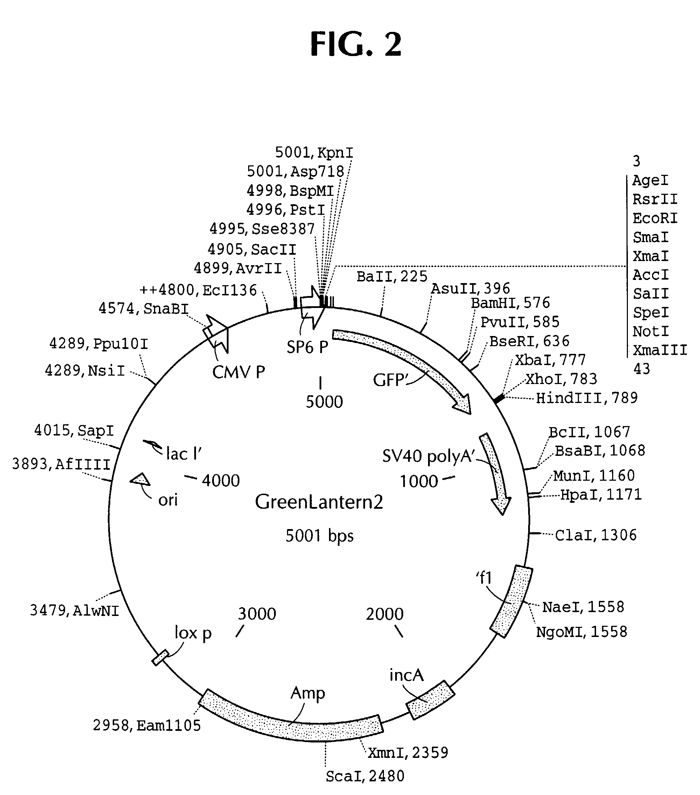

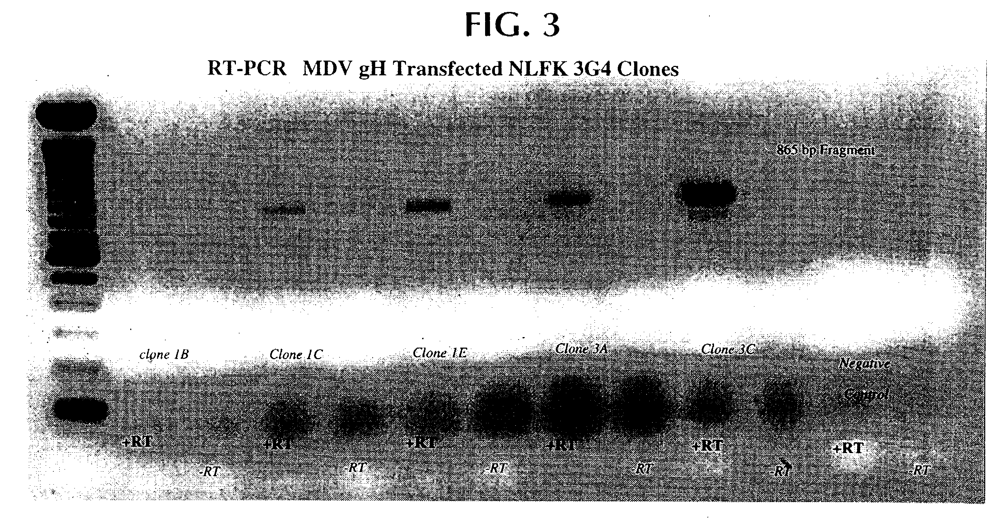

The construct pCR3.1gH was introduced into the NLFK 3G4 cells by lipofection, using liposome reagent available from Qiagen, U.S.A., SuperFect Catalog Number 301305). Pools of stable transfectants were generated using 300 µg/ml G418 ( available from Gibco BRL, U.S.A., Catalog Number 10131-019) selection medium (available from Optimem I Gibco BRL, U.S.A., Catalog Number 11058021 and 1% Fetal Bovine Serum (avaiable from Gibco BRL, U.S.A., Catalog Number 16000-036). Transfected cells express the neomycin resistance gene and will be unaffected by G418 which is toxic to the NLFK 3KG4 cells. Total RNA was isolated from the pools of G418 resistant cells using the Biotex Ultraspec RNA Isolation Systerm, Catalog Number BL-10050. Total RNA (lug per sample) from the pools was tested and found positive by reverse transcriptase polymerase chain reaction (RT-PCR) ( using SuperScript Preamplification System available from Gibco BRL, U.S. A., Catalog Number 18089-011, PCR Reagent System available from Gibco BRL, U.S.A., Catalog Number 10198-018) indicating that MDVgH mRNA was being expressed. Single cell clones were obtained by limiting dilution. After expansion in G418 selection medium (as described above), total RNA from the cloned cells was isolated and tested as before by RT-PCR (see Figure 3). Frozen cell stocks were prepared from the resulting RT-PCR positive cloned cells. In accordance with Figure 2, one µg total RNA was tested by RT-PCT. Samples were tested in matched pairs with (+RT) and without the addition of reverse transcriptase (-RT). A negative control was included containing all reagents but no RNA template to confirm reagents were not compromised. In four of the clones shown, (1C, 1E, 3A and 3C) the 865 bp gH fragment was amplified in the +RT lane. For each sample the -RT lane is negative indicating that the positive signal is from MDV gH mRNA and not contaminating DNA.

### Example 8

### Production of Recombinant MDV Vector

A plasmid construct is prepared which has a detectable marker. The marker gene is surrounded by the 5' and 3' flanking regions of the gH gene, which are used for homologous recombination. The 5' flanking region of MDV-1 gH was cloned by PCR (Upper PCR primer: 5'-GGGAT GCATT ACGGT CTCTG AACAA G-3', Lower PCR primer: 5'-GGGAT GCATA AGGAC GCCA ATTAC TATCT-3') and put into the blunt-end treated Nsi1 site (as described below) of pGreenLanern-2 plasmid, available from GIBCO-Life Technologies, U.S.A (see Figure 2). The 5' flanking region of MDV-1 gH is available from GeneBank, U.S.A, contains the TK gene at position 894 to 1925. The pGreenLantern-2 plasmid has the gene encoding for the jellyfish green fluorescence protein (gfp) which is used as the marker protein in the process of generating gH-deleted recombinant virus. The Nsi1 site of pGreeLantem-2, upstream of the CMV promoter for the green fluorescence protein gene, is treated with T4 polymerase to create a blunt-end for ligating with the 5' gH flanking region containing Pme 1 ends.

A plasmid was obtained from Dr. Meihan Nonoyama of the Tampa Bay Research Institute FL, U.S.A, containing the MDV-1 BamH1 fragment "F", which includes the gH 3' flanking region [Fukuchi et al., J. Virol. 102-109, (1984)]. The DNA sequence of about a 2.4 kb region (referred to herein as the 3" flanking region") is shown in SEQ ID No: 2. The MDV-1 gH 3' flanking region was obtained by PCR cloning (Upper primer: 5'-AAGAT TTTTC CCAAG TCC-3'; Lower primer: 5' -TCGTC GAATA ATGTG ATC-3') and cloned into the above mentioned pGreenLantern-2 plasmid which has the MDV-1 5' flanking region inserted at the Nsi1 site. The 3' flanking region was inserted into the Nael site, downstream of the whole green fluorescence protein gene.

This plasmid which has green fluorescence protein gene flanked by the 5' and 3' flanking regions of MDV-1 gH is transfected into the gH-producing 3G4 cell line infected with MDV-1. Green virus plaques are selected under the ultaviolet microscope and purified from parental MDV-1 viruses. The resulting new recombinant virus has the gH gene deleted and replaced by the gene encoding for the green fluorescence protein.

In a second recombination step to remove the green fluorescence protein gene from the gH gene deleted virus, on can optionally insert heterologous genes from viruses other than MDV, such as, for example, the chicken interferon gene, Newcastle disease virus, or infection bursal disease viruses etc., which can replace the green fluorescence protein gene. The presence of white virus plaques in contrast to parental green plaques, contain the new recombinant MDV-1 virus in which the gH gene is deleted and the green fluorescence protein gene removed.

## Claims

1. A method for producing MDV which comprises:
(a) infecting or transfecting a continuous mammalian cell line with MDV; and
(b) culturing the continuous mammalian cell line infected or transfected with MDV.

2. The method of claim 1 wherein the MDV is a recombinant molecule comprising the nucleic acid sequence of MDV or a fragment(s) thereof.

3. The method of claim 1 wherein the MDV is a recombinant molecule comprising the nucleic sequence of MDV and at least one heterologous gene or fragment(s) thereof inserted into said nucleic acid sequence of MDV.

4. The method of claim 1 wherein the MDV is a recombinant molecule comprising the nucleic acid sequence of MDV from which one or more genes, regulatory genetic elements, or fragment(s) thereof has been deleted.

5. The method of claim 4 wherein the deleted gene is essential for replication.

6. The method of claim 5 wherein the deleted gene is gH or a fragment(s) thereof.

7. The method of claim 1 wherein the MDV is selected from the group consisting of serotype 1, serotype 2 and serotype 3, taken singly or in any combination thereof.

8. The method of claim 1 wherein the MDV is a virus used for the preparation of a vaccine capable of inducing protection against disease in avians.

9. The method of claim 1 wherein the cell line is a feline kidney cell line.

10. The method of claim 9 wherein the MDV is MDV-1 strain Md5.

11. The method of claim 10 wherein the cell line is ATCC CRL-12336.

12. A continuous mammalian cell line infected or transfected with MDV.

13. The cell line of claim 12 wherein the MDV is a recombinant molecule comprising the nucleic acid sequence of MDV or a fragment(s) thereof.

14. The cell line of claim 12 wherein the MDV is a recombinant molecule comprising the nucleic acid sequence of MDV or a fragment(s) thereof and having at least one heterologous gene or fragment(s) thereof inserted into said nucleic acid sequence of MDV.

15. The cell line of claim 12 wherein the MDV is a recombinant molecule comprising the nucleic of MDV from which one or more genes, regulatory genetic elements, or fragment(s) thereof has been deleted.

16. The cell line of claim 15 wherein the deleted gene is essential for replication.

17. The cell line of claim 16 wherein the deleted gene is gH or a fragment(s) thereof.

18. The cell line of claim 12 wherein the MDV is selected from the group consisting of serotype 1, serotype 2 and serotype 3, taken singly or in any combination thereof.

19. The cell line of claim 12 wherein the MDV is a virus used for the preparation of a vaccine capable of inducing protection against disease in avians.

20. The cell line of claim 12 wherein the cell line is a feline kidney cell line infected or transfected with MDV.

21. The cell line of claim 20 wherein the MDV is MDV-1 strain Md5.

22. The cell line of claim 21 wherein the cell line is ATCC CRL-12336.

23. A method for producing a MDV vaccine comprising culturing a continuous mammalian cell line infected or transfected with MDV and harvesting cell culture components therefrom.

24. A method for producing a continuous mammalian cell line infected or transfected with MDV which comprises:
(a) culturing continuous mammalian cells with MDV; and
(b) propagating the MDV infected or transfected mammalian cells to produce the cell line.

25. A vaccine comprising MDV produced by the method of claim 1.

26. The vaccine of claim 25 wherein the MDV is a recombinant molecule comprising the nucleic acid sequence of MDV or a fragment(s) thereof.

27. The vaccine of claim 25 wherein the MDV is a recombinant molecule comprising the nucleic acid sequence of MDV and at least one heterologous gene or a fragment(s) thereof inserted into said nucleic acid sequence of MDV.

28. The vaccine of claim 25 wherein the MDV is a recombinant molecule comprising the nucleic acid sequence of MDV from which one or more genes, regulatory genetic elements, or fragment(s) thereof has been deleted.

29. The vaccine of claim 28 wherein the deleted gene is gH or a fragment(s) thereof.

30. The vaccine of claim 25 wherein the MDV is selected from the group consisting of serotype 1, serotype 2 and serotype 3, taken either singly or in any combination thereof.

31. The vaccine of claim 25 wherein the cell line is a feline kidney cell line infected or transfected with MDV.

32. The vaccine of claim 31 wherein the MDV is MDV-1 strain Md5.

33. The vaccine of claim 32 wherein the cell line is ATCC CRL-12336.

34. A recombinant MDV molecule produced by the method of claim 1.

35. The recombinant MDV of claim 34 wherein the recombinant MDV comprises the nucleic acid sequence of MDV from which one or more genes have been deleted.

36. The recombinant MDV of claim 35 wherein one or more of the deleted genes is a gene essential for replication.

37. The recombinant MDV of claim 36 wherein the deleted gene is gH or a fragment(s) thereof.

38. The recombinant MDV of claim 34 wherein the cell line is a feline kidney cell line infected or transfected with MDV.

39. The recombinant MDV of claim 38 wherein the MDV is MDV-1 strain Md5.

40. The recombinant MDV of claim 39 wherein the cell line is ATCC CRL-12336.

41. A MDV vector produced using the method of claim 1.

42. The MDV vector of claim 41 wherein the MDV vector has a 3' flanking region comprising a nucleic acid sequence as described in SEQ ID NO: 2.

43. The MDV vector of claim 42 wherein the MDV vector has a 5' flanking region comprising a nucleic acid sequence as described in SEQ ID NO: 1.

44. A method for protecting animals against disease by admininstering to such animals a vaccine comprising MDV produced by the method of claim 1.

45. The method of claim 44 wherein the animal is avian.
